# EUROPEAN PATENT SPECIFICATION

(11) **EP 2 672 954 B1**
(45) Date of publication and mention of the grant of the patent: **19.04.2017**
(21) Application number: 12703792.7
(22) Date of filing: 10.02.2012
(51) Int. Cl.: A61K 8/99, A61Q 17/04

(54) **BIOTECHNOLOGICALLY DERIVED ACTIVE IN COSMETIC COMPOSITIONS USEFUL FOR PROTECTING THE SKIN FROM DAMAGES INDUCED OR PRODUCED BY INFRARED-RADIATIONS**
VERWENDUNG EINER BIOTECHNOLOGISCH ERHÄLTLICHEN SUBSTANZ IN KOSMETISCHEN ZUSAMMENSETZUNGEN ZUM SCHUTZ VOR HAUTSCHÄDEN DIE DURCH INFRAROTSTRAHLUNG HERVORGERUFEN WERDEN
PRINCIPE ACTIF DÉRIVÉ DE LA BIOTECHNOLOGIE, DANS DES COMPOSITIONS COSMÉTIQUES POUR PROTÉGER LA PEAU DE LÉSIONS INDUITES OU PRODUITES PAR DES RAYONNEMENTS INFRAROUGES

(30) Priority: 11.02.2011 EP 11382038
(43) Date of publication of application: 18.12.2013
(73) Proprietor: Laboratorios Cinfa, S.A., 31620 Huarte (ES)
(72) Inventor: ELIZARI GALAR, Maialen, E-31620 Gorraiz (ES); GARRE CONTRERAS, Aurora Del Carmen, E-31110 Noain (ES); DE CASTELLARNAU CASTELLÀ, Conxita, E-08029 Barcelona (ES)
(74) Representative: ZBM Patents - Zea, Barlocci & Markvardsen
(86) International application number: PCT/EP2012/052282
(87) International publication number: WO 2012/107550

(56) References cited:
- FR-A1- 2 920 306
- FR-A1- 2 937 534
- JP-A- 2000 229 827
- US-A1- 2002 168 388
- DATABASE GNPD [Online] Mintel; 1 March 2010 (2010-03-01), "Repair Extract Base", XP002645624, Database accession no. 1283274
- SCHROEDER P ET AL: "Prevention of infrared-A radiation mediated detrimental effects in human skin.", SKIN THERAPY LETTER JUN 2009 LNKD- PUBMED:19609472, vol. 14, no. 5, June 2009 (2009-06), pages 4-5, XP002645625, ISSN: 1201-5989

## Description

The invention relates to the field of cosmetics and/or dermatological products, more particularly those intended for skin protection against solar radiation. In particular, the invention relates to the use of a biotechnologically derived active for the protection of the skin against cellular damage caused by infrared radiation, as well as to cosmetic compositions comprising it.

### BACKGROUND ART

Solar radiation is well known to damage human skin, for example by causing premature skin ageing (i.e. photoaging). This damage does not result from ultraviolet (UV) radiation alone, but also from longer wavelengths, in particular near-infrared radiation (IRA radiation, 760-1440 nm). Since natural sunlight is polychromatic, its ultimate effects on the human skin are the result of not only the action of each wavelength separately, but also interactions among the many wavelengths, including UV, visible light, and infrared (IR).

Sunscreens are the main protectors against solar radiation. They can reflect, absorb or scatter sunlight, protecting the skin from the harmful effects of sun. Solar photo-protective treatments protect and prevent skin damage resulting from solar radiation. The classic photo protectors only protect against UVB and UVA.

UV light is responsible for collagen fibers destruction and for abnormal elastic fibers production. It also promotes alterations on the dermal microvascularization. Depending on ray's intensity, the skin may suffer degeneration in its structure and abnormalities in its normal pigmentation process. Besides, the free radicals formation has an important role in the photoaging.

UVB light causes DNA damage but also inhibits DNA repair. As a consequence of the DNA damage up regulation of IL-10 occurs, which, in turn leads to inhibition of the formation of IL-12. IL-12 is one of the major players involved in orchestrating immune responses. Keeping the IL10/IL12 balance is a prerequisite for counteracting photoaging and chronic photo damage. In particular, intensive exposure of the skin to UV radiation leads to DNA mutagenic base modifications, in particular, to a thymidine dimerization by cycloaddition between two adjacent thymidine elements of DNA. This change in the structure of the nucleic acid can lead to either the death of the cell or to inheritable cell damage. The natural DNA repair mechanism of the cells based on an enzimatically controlled reaction sequence in order to cut the thymidine dimers out of the DNA strand and to replace these by thymidine monomers possesses only a limited capacity.

Therefore, an effective solar protector must prevent from burnings but also minimize the skin damage due to an accumulation of the received radiation. There are mainly three types of filters employed in photo protection: physical, chemical and biological filters. Physical filters are inorganic or inert powders of small particles around 180-190 nm of diameter, composed by TiO₂, ZnO, ferrous oxide, MgO, mica or talc. They act reflexing the solar radiations independently of its wave length. Chemical filters are synthetic chemicals that act by absorbing solar radiation. They function as chromophores absorbing the energy carried by an incident photon, then they return to its initial state and release excess energy as heat (negligible), fluorescent radiation or chemical transformation in a potentially reactive isomer photoproduct. Biological filters are substances with antioxidant activity that, when applied topically, reduce oxidative stress induced by UV radiation. They enhance the protection of traditional sunscreens decreasing cell damage that might be the cause of photoaging and skin cancer. They are vitamins (A, C or E), flavonoids (Fe chelator) and certain oligoelements (enzyme activity). Antioxidants are molecules that act by neutralizing some of the resulting free radicals which are generated by UV damage and lead to breakdown of collagen, and consequently antioxidant lessen or prevent the signs of photoaging skin.

European patent application EP43128 discloses the use of a microorganism lysate such as a lysate of *Bifidobacterium longum*, for promoting the repair of DNA in skin cells damaged by exposure to ultraviolet radiation. It was shown to inhibit the release of immunosuppressant IL-10, which simultaneously prevents the inhibition of IL-12, and allows increasing the body's own DNA repair capacity of the skin cells.

In order to achieve complete protection against the cumulative detrimental effects from sun exposure, topical strategies must shield against the range of solar wavelengths that can damage the skin. Importantly, the harm sustained by the skin is not limited to that caused by the ultraviolet (UV) portion of the light spectrum, but also includes the adverse effects inflicted by near infrared energy.

Infrarred A radiation (IRA radiation) accounts for more than one third of the solar energy that reaches human skin. This IRA radiation is considered the ultimate threat that comes from the sun due to their ability to penetrate to the deepest layer of skin. The IRB radiation and IRC are responsible for its thermal effect. While infrared radiation of longer wavelengths (IRB and IRC) does not penetrate deeply into the skin, more than 65% of the shorter wavelength (IRA) reaches the dermis.

Until relatively recently it was believed that the effect of IRA on skin was thermal producing vasodilatation and redness, but recent studies show that penetrating deeply produces a direct attack on cellular mitochondria, responsible for the cell's energy supply, producing an increase of free radicals, especially reactive oxygen species (ROS), which produce deleterious biological effects on skin cells. It has been stated that IRA has a considerably contribution in the sunburns, the DNA damage and premature skin aging. It also seems that skin cancer is not only consequence of the exposure to UV radiation, but the result of the sunburns by oxidative stress (cf. Zastrow L. et al. "The Missing Link - Light-Induced (280-1,600nm) Free Radical Formation in Human Skin ", Skin Pharmacol and Physiol 2009, vol. 22, pp. 31-44, or Darvin M.E. et al. "Radical Production by Infrared A Irradiation in Human Tissue", Skin Pharmacol and Physiol 2010, vol. 23, pp. 40-46).

When free radicals accumulate in the cell, as a consequence of IRA induced mitochondrial oxidative stress response, an increase in the expression of matrix metalloproteinases (MMPs) and reduced collagen and elastin synthesis occurs. Thus, IRA radiation alters the collagen equilibrium of the dermal extracellular matrix in at least two ways: (a) by leading to an increased expression of the collagen-degrading enzyme matrix metalloproteinase-1(MMP-1), and (b) by decreasing the de novo synthesis of the collagen itself. IRA radiation exposure therefore induces similar biological effects to UV radiation, but the underlying mechanisms are substantially different, specifically, the cellular response to IRA irradiation involves the mitochondrial electron transport chain and a signaling response, which generate ROS. P. Schroeder et al., in "Cellular response to infrared radiation involves retrograde mitochondrial signaling", Free Radic. Biol. Med. 2007, vol. 43, pp. 128-135 explains that mitochondria-derived ROS are not involved in UVA or in UVB radiation-induced MMP-1 expression because mitochondrial targeted coenzyme Q10 or manipulation of mitochondrial electron transport chain activity did not affect these responses. Several studies indicate that the main inductor of ROS generation and cellular accumulation is the increase of the intracellular temperature caused by IRA radiation.

Thus, in addition to solar filters to block the effects of UV or molecules that help to repair the damaged DNA due to UV radiation, novel strategies to block IR- and heat-damages, in particular, IR- and heat- induced skin photoaging need to be developed to prevent skin photoaging more completely, in particular, to prevent or delay the cutaneous signs of photoaging. Antioxidants such as carotenoids, flavonoids or vitamins, are effective against oxidative stress, but alone are not effective against IRA.

EP2233127 discloses the use of several antioxidants for the preparation of a pharmaceutical or a cosmetic composition to the protection of the skin against damage by infrared radiation. Among them the following are cited: N-acetylcysteine, vitamin E, coffee-acidic, ester of the coffee-acidic, [beta] - carotene, Emblica, vitamin of C-derivatives, becoming green excerpt, noble white excerpt, Lutein, Lycopen, grape/cluster core excerpt, Chicory extract, Curcumin, Silymarin, Apigenin and Resveratrol.

WO 98/01107 discloses a cosmetic composition for skin moisturizing and protection against UV and IR radiation comprising specific ranges from several components. Among these components is a mineral pigment, which is basically titanium dioxide micronized, which is cited as exerting an IR protective function.

Thus, from what it is known in the art, it is derived that there remains a need to provide new molecules capable of interfering with and/or preventing the deleterious effects of infrared A (IRA) radiation.

### SUMMARY OF THE INVENTION

The inventors have found, unexpectedly, that a lysate of the genus *Bifidobacterium* species, in particular *Bifidobacterium longum*, prove to be efficient in preventing and/or treating photoaging of skin induced or produced by infrared radiation. This founding is based on the demonstration by the inventors of the capacity of a lysate of *Bifidobacterium longum* to diminish ROS generation directly induced by the temperature after different exposures time and to inhibit the MMP-1 production. It shows an antioxidant effect against IRA radiation. As a consequence it has the capacity of protecting against cellular damage produced by IRA radiation, which means the protection of the skin against photoaging.

A specific lysate of *Bifidobacterium longum* is sold under the name Repair Complex CLR by the company K. Richter GmbH. The Repair Complex relates to a lysate registered under the INCI name: *Bifidat ferment Lysate*, under the EINECS name: *Bifidobacterium longum*, under the EINECS No.: 306-168-4 and under CAS No.: 96507-89-0. It is an extract from *Bifidobacteria*, containing metabolite products, cytoplasma fractions, cell wall constituents, as well as polysaccharide complexes. It is sold preserved in paraben (Repair Complex) or paraben free (Repair Complex PF, RCPF).

As far as the inventors are aware, this efficacy of a lysate of a microorganism of the genus *Bifidobacterium* species, in particular of a lysate of *bifidobacterium longum*, and more in particular the product Repair Complex, in the protection against skin cells damage produced by IRA radiation have never been described.

A lysate of *bifidobacterium longum* is known from EP43128, but only for the purposes of promoting the repair of the DNA of the skin cells damaged by exposure to UV radiation. Other uses of the product are already known. For instance, WO2009/053564 (or FR2920306 from the same family) describes-its use to prevent or treat skin signs of chronological aging and disorders caused by oxidative stress generated by an environmental oxidant such as oxygen, ozone and / or oxides of nitrogen and sulfur, as well as a composition comprising a lysate of bifidobacterium. The US patent application US20090060962 describes its use for treating and/or preventing dryness and/or associated disorders of a keratinous substance. It is also described its use in preventing and/or reducing wrinkles related to cutaneous drying. The US patent application US20090068610 describes the use of the lysate of *bifidobacterium longum* Repair Complex CLR for preventing and/or treating skins disorders liable to be manifested on skin termed as sensitive skin.

Finally, other compositions comprising a lysate of bifidobacterium are also known in the art. FR2937534 discloses a cosmetic and/or dermatology composition, including at least, in a physiologically acceptable medium, an orchid extract, biotin or biotin derivative and a lysate of bifidobacterium.

However, none of these documents discloses the application of a microorganism of the genus *Bifidobacterium* species, and even less a lysate according to the invention, as active with protective effect against skin cells damage produced by the IRA radiation.

Thus, according to a first aspect, the present invention provides an effective amount of a lysate of at least one microorganism of the genus *Bifidobacterium* species, in particular *Bifidobacterium longum*, for use in the prevention and/or treatment of the cutaneous signs of photoaging induced or produced by infrared radiation.

This biological substance can be combined with physical, chemical, organomineral, and/or biological filters in order to provide a photo protector of a broad-spectrum which avoids photoaging, allowing the exposition to solar radiation with less risk. It is disclosed a composition, preferably a cosmetic composition. comprising a lysate of at least one microorganism of the genus *Bifidobacterium* species, and at least a filter against solar radiation selected from the group consisting of physical, chemical, organ mineral, biological, and combinations thereof, preferably in an amount of between 29-50% by weight of the total composition, together with cosmetically acceptable excipients or carriers appropriate for topical application to a person's skin.

By the application of these cosmetic compositions a complete protection against the cumulative detrimental effects from sun exposure, is achieved retarding the light-caused aging process of the skin, which imparts to these compositions a high practical value from the cosmetic viewpoint. Accordingly, a third aspect of the present invention relates to an effective amount of a lysate as defined above, in combination with at least a filter against solar radiation selected from the group consisting of physical, chemical, organ mineral, biological filter, and combinations thereof in an amount of between 29-50% by weight of the total composition, together with cosmetically acceptable excipients or carriers appropriate for topical application to a person's skin, for use in the prevention and/or treatment of the cutaneous signs of photoaging induced or produced by UV and IR radiation, in particular, IRA radiation,

It is disclosed a method, in particular a cosmetic method, for preventing and/or treating skin cells from damage by exposure to IR radiation, in particular IRA radiation, comprising administering an effective amount of a lysate of at least one microorganism of the genus *Bifidobacterium* species, in particular *Bifidobacterium longum*, together with cosmetically acceptable carriers or excipients appropriate for topical application to a person's skin, to a subject in need of such prevention and/or treatment.

It is also considered as part of the invention a lysate of at least one microorganism of the genus *Bifidobacterium* species, in particular *Bifidobacterium longum*, for use in the prevention and/or treatment of skin cells damage produced by IR radiation, in particular, IRA radiation. The skin cells damage is the damage that is done to the skin from prolonged exposure, to IRA radiation. More particularly, the skin cell damage is photoaging induced or produced by IR radiation, in particular IRA radiation.

It is also part of the invention the provision a lysate of at least one microorganism of the genus *Bifidobacterium* species, in combination with at least a filter against solar radiation selected from the group consisting of physical, chemical, organomineral, biological filters, and combinations thereof, for use in the prevention and/or treatment of skin cells damage produced by IR radiation, in particular IRA radiation and UV radiation. More particularly, the skin cell damage is photoaging induced or produced by IR radiation, in particular IRA radiation, and UV radiation. In that case the skin cells damage is the damage that is done to the skin from prolonged exposure, to IR radiation and UV radiation.

In a particular embodiment, the prevention and/or treatment comprises diminishing ROS generation directly induced by IR radiation, in particular IRA radiation. It comprises administering an effective amount of a lysate of at least one microorganism of the genus *Bifidobacterium* species, in particular *Bifidobacterium longum*, together with cosmetically acceptable carriers or excipients appropriate for topical application to a person's skin, to a subject in need of such prevention and/or treatment.

In a particular embodiment, the prevention and/or treatment comprisesinhibiting the MMP-1 production induced by IR radiation, in particular IRA radiation. It comprises administering an effective amount of a lysate of at least one microorganism of the genus *bifidobacterium* species, in particular *Bifidobacterium longum*, together with cosmetically acceptable carriers or excipients appropriate for topical application to a person's skin, to a subject in need of such prevention and/or treatment.

### BRIEF DESCRIPTION OF THE DRAWINGS

FIG. 1A shows the overall effect of the controls at different temperatures (37 ° C and 42 ° C) in the measure of the direct effect of temperature increase in intracellular ROS production in human fibroblasts and the comparison with oxidative stimulus tert-butyl-hidroperoxide (TBHP). "t" means incubation time, "ctrl" means control" and "c probe" means the ROS sensitive probe carboxy-H₂ DCFDA , C400 from Molecular Probes.
FIG. 1B shows the relative increase of ROS by either the thermal stress and or the oxidative stimulus (TBHP) in human fibroblast after incubating the cells at 42 °C over its value at 37 °C. "t" means incubation time, "ctrl" means control", "X-fold ROS increment" means times of ROS increment vs. control.
FIG. 2A shows the measurement of intracellular ROS production in human fibroblasts incubated at 42 °C for 60 min. in response to several concentrations of RCPF and Trolox (100µM).. "Ctr" means the control, RCPF means the lysate of *bifidobacterium longum* named Repair Complex PF. Trolox is a reference antioxidant and "c probe" means the ROS sensitive probe carboxy-H₂ DCFDA, C400 from Molecular Probes.
FIG. 2B shows the measurement of intracellular ROS production in human fibroblasts incubated at 42 °C for 120 min. in response to several concentrations of RCPF and Trolox.
FIG. 2C shows the measurement of intracellular ROS production in human fibroblasts incubated at 42 °C for 180 min. in response to several concentrations of RCPF and Trolox.
FIG. 2D shows the measurement of intracellular ROS production in human fibroblasts incubated at 42 °C for 240 min. in response to several concentrations of RCPF and Trolox.
FIG. 3 shows a comparison between the measurement of intracellular ROS production in human fibroblasts incubated at 42 °C for 60, 120, 180 and 240 min. in response to several concentrations of RCPF and Trolox.
FIG. 4 shows the effect of RCPF over the MMP-1 synthesis in human fibroblasts incubated at 37°C and at 42 °C for the 1 h, 2h, 3h, and 4h and subsequently inflamed with interleukin 1β before adding or not (C medium) the studied products. The "t" means time of treatment (24 h) post incubation at 42 °C.
FIG. 5A shows the effect of RCPF over the MMP-1 synthesis in human fibroblasts incubated at 42 °C for 1 h. IL-1β 5ng/ml is used as a positive inducer of MMP-1 synthesis in these assays.
FIG. 5B shows the effect of RCPF over the MMP-1 synthesis in human fibroblasts incubated at 42 °C for 2h. IL-1β 5ng/ml is used as a positive inducer of MMP-1 synthesis in these assays.
FIG. 5C shows the effect of RCPF over the MMP-1 synthesis in human fibroblasts incubated at 42 °C for 3h. IL-1β 5ng/ml is used as a positive inducer of MMP-1 synthesis in these assays.
FIG. 5D shows the effect of RCPF over the MMP-1 synthesis in human fibroblasts incubated at 42 °C for 4h. IL-1β 5ng/ml is used as a positive inducer of MMP-1 synthesis in these assays.

### DETAILED DESCRIPTION OF THE INVENTION

For the purposes of the present disclosure, the term "photoaging" relates to the effects of ultraviolet UV light exposure on skin and/or IR light exposure on skin associated with the formation of coarse wrinkles, uneven skin pigmentation, loss of skin elasticity, a disturbance of skin barrier functions, or a combination thereof. Thus, cutaneous signs of photoaging include changes in pigmentation (mottled pigmentation), sallowness, deep wrinkling, dryness, telangiectasia, premalignant lesions, laxity, atrophy, leathery appearance, elastosis (a coarse, yellow, cobblestoned effect of the skin), or actinic purpura (easy bruising related to vascular wall fragility in the dermis).

For the purposes of the present disclosure, the term "effective amount" means an amount that is sufficient to obtain the expected effect.

For the purposes of the present disclosure, the term "preventing" means reducing the risk of manifestation of a phenomenon.

For the purposes of the present disclosure the term "treating" means compensating for a physiological dysfunction and more generally reducing or even eliminating an undesirable disorder, the manifestation of which is especially a consequence of this dysfunction.

For the purpose of the invention, the term "cosmetic" is intended to denote a use intended, principally, to provide and aesthetic and/or comfort effect, in particular, to ameliorate the appearance of the skin, specifically the properties of the skin.

For the purpose of the invention, the term "cosmetically acceptable excipients or carriers" refers to that excipients or carriers suitable for use in contact with human skin without undue toxicity, incompatibility, instability, allergic response, among others.

All the percentages mentioned herein are in weight.

As mentioned above, an aspect of the present invention is the provision of an effective amount of a lysate of at least a genus of bifidobacterium species, in particular, bifidobacterium longum for use in the prevention and/or treatment of the cutaneous signs of photoaging induced or produced by IR radiation, in particular IRA radiation. It can also be formulated as a lysate of at least one microorganism of the genus *Bifidobacterium* species, for use in the prevention and/or treatment of the cutaneous signs of photoaging induced or produced by IR radiation, in particular, IRA radiation, and in particular, wherein the use is cosmetic, i.e. for cosmetic purposes. In a preferred embodiment of this aspect the lysate of at least one microorganism of the genus *Bifidobacterium* species is forming part of a cosmetic composition. It is disclosed a cosmetic method for preventing and/or treating the cutaneous signs of photoaging induced or produced by IR radiation, in particular, IRA radiation, comprising administering an effective amount of a lysate of at least one microorganism of the genus *Bifidobacterium* species, in particular *Bifidobacterium longum*, together with cosmetically acceptable carriers or excipients appropriate for topical application to a person's skin, to a subject in need of such prevention and/or treatment.

In a preferred embodiment, the use is for prevention of the cutaneous signs of photoaging.

For the purposes of the invention, a "lysate" conventionally denotes a material obtained after the destruction or dissolution of biological cells via a phenomenon known as cell lysis, thus giving rise to the release of the intracellular biological constituents naturally contained in the cells of the microorganism under consideration. For the purposes of the present invention, the term "lysate" is used without preference to denote the whole lysate obtained via lysis of the microorganism under consideration or only a fraction thereof.

The lysate used is thus totally or partially formed from the intracellular biological constituents and from the constituents of the cell walls and membranes. This cell lysis may be accomplished via various techniques, such as an osmotic shock, a heat shock, via ultrasonication, or alternatively under a mechanical stress of centrifugation type. More particularly, this cell lysate may be obtained according to the technology described in US 4464362, and EP 43128. The microorganism of the *Bifidobacterium* species type considered can be cultivated anaerobically in a suitable culture medium, for example according to the conditions described in US 4464362, and EP 43128. When the stationary phase of development is reached, the culture medium can be inactivated by pasteurization, for example, at a temperature of 60 to 65 °C for 30 min. The microorganisms are then collected by a conventional separation technique, for example membrane filtration, centrifugation and resuspension in a sterile physiological NaCl solution. The lysate can be obtained by ultrasonic disintegration of said medium in order to release its cytoplasmatic fractions, the fragments of cell wall and the products resulting from metabolism. Then all the components in their natural distribution are stabilized in a weekly acid aqueous solution.

In this way a lysate is generally obtained that has a concentration of the order of 0.1 to 50%, in particular from 1 to 20% and notably about 5% by weight of active substance/s relatively to its total weight.

The lysate can be used in various forms, in the form of a solution or in a pulvurent form.

The microorganism belonging to the genus *Bifidobacterium* species is selected from the species: *Bifidobacterium longum, Bifidobacterium bifidum, Bifidobacterium breve, Bifidobacterium animalis, Bifidobacterium lactis, Bifidobacterium infantis, Bifidobacterium adolescentis, Bifidobacterium pseudocatemulation,* and mixtures thereof.

The preferred species is *Bifidobacterium longum,* which results to be particularly suitable for the purposes of the invention.

The product sold under the name Repair Complex CLR® is included in the context of the disclosure. According to the information'supplier Repair Complex CLR PF is obtained by fermentation of the species "longum". After growth is completed, the bacteria are disintegrated by means of ultrasound thus releasing the cytoplasm fractions and cell wall constituents. After cell disintegration, no fraction whatsover are isolated, thus ensuring the presence of all constituent in their natural distribution in the product Repair Complex CLR PF. Both, the one form CLR commercialized preserved in parabens or the one commercialized as paraben free can be used for the purposes of the present invention.

The lysate of *Bifidobacterium*, in particular of *bifidobacterium longum*, can be formulated in a composition according to the present invention at a rate from between 0.1-10% in weight based on the total weight of the composition. Preferably, at a rate from 0.5-5% in weight based on the total weight of the composition. More preferably, at a rate of 0.5-4% in weight based on the total weight of the composition. Even more preferably, at a rate of 0.5-3% in weight based on the total weight of the composition. Even still more preferably, at a rate of 0.5-1 % in weight based on the total weight of the composition.

All percentages mentioned herein are percentages by weight unless otherwise indicated.

It may also be desirable to include one or more sunscreens in a composition comprising the lysate of the genus bifidobacterium species, preferably *Bifidobacterium longum*. This lysate can be combined with UVA or UVB filters, in order to provide a photo protector of a broad-spectrum. The cosmetic composition can be used for the prevention and/or treatment of the cutaneous signs of photoaging induced or produced by UV and IR radiations, in particular UVA, UVB and IRA radiations. As mentioned above, cutaneous signs of photoaging include changes in pigmentation, sallowness, deep wrinkling, dryness, telangiectasia, premalignant lesions, laxity, atrophy, leathery appearance, elastosis, or actinic purpura, among others.

It is considered part of the invention the provision of an effective amount of a lysate of at least a genus of bifidobacterium species, in particular, bifidobacterium longum, in combination with at least a filter against solar radiation selected from the group consisting of physical, chemical, organomineral, biological filters, and combinations thereof, in an amount of between 29-50% by weight of the total composition, together with cosmetically acceptable excipients or carriers appropriate for topical application to a person's skin. for use in the prevention and/or treatment of the cutaneous signs of photoaging induced or produced by UV and IR radiations, in particular UVA, UVB and IRA radiations, and in particular, wherein the use is cosmetic.. That means that the lysate of at least one microorganism of the genus *Bifidobacterium* species, in combination with at least a filter against solar radiation are forming part of a cosmetic composition. It is disclosed a cosmetic method for preventing and/or treating the cutaneous signs of photoaging induced or produced by UV and IR radiations, in particular UVA, UVB and IRA radiations comprising administering an effective amount of a lysate of at least one microorganism of the genus *Bifidobacterium* species, in particular *Bifidobacterium longum*, together with cosmetically acceptable carriers or excipients appropriate for topical application to a person's skin, to a subject in need of such prevention and/or treatment.

Advantageously, inclusion of sunscreens in a composition comprising the lysate of at least one microorganism of the genus *Bifidobacterium* species provide additional protection to skin exposed to solar radiation.
It is disclosed a composition consisting of a lysate of at least one microorganism of the genus *bifidobacterium* species, and at least a filter against solar radiation selected from the group consisting of physical, chemical, organomineral, biological, and combinations thereof, preferably in an amount of between 29-50% by weight of the total composition, together with cosmetically acceptable excipients or carriers appropriate for topical application to a person's skin.

In the composition of the invention, pharmaceutically acceptable excipients or carriers can also be used.

The organic filters, for example, can be selected from those approved by the Council of the European Communities (revised text of European Directive 76/768/EEC consolidated version Annex VII pages 121-126, published on 24.04.2008). The inorganic filters can be selected from a group that includes: metallic oxides as pigments, nanopigments, treated and untreated, such as the dioxide of titanium (amorphous or crystalline), iron, zinc, zirconium or cerium.

If present, the amount of organic and inorganic filters in the cosmetic composition disclosed herein may range from about 0.1% to 50%. Generally, the amount of organic and inorganic filters in the cosmetic composition is of between 29% and 50% by weight of the total composition. In a preferred embodiment, the amount of organic and inorganic filters in the cosmetic composition is of between 29% and 35% by weight of the total composition.

Thus, the composition may comprise one or more UVA screens. The term "UVA screen" means a chemical compound that blocks UV radiation in the wave-length of about 320-440 nm. Examples of UVA sunscreen are 2-hydroxy-4-methoxybenzofenone (oxibenzone, INCI name: benzophenone-3); 3,3'-(1,4-phenylendimethylen) bis [7,7-dimethyl-2-oxo-bicyclo-(2,2,1) hept-1-yl methansulfónic] or their cosmetically acceptable salts (INCI name: terephthalydene dicamphor sulfonic acid); 1-(4-tert-butyl-phenyl)-3-(4-methoxyphenyl)propan-1,3-dione (INCI name: butyl methoxydibenzoyl methane); phenol,2-(2H-benzotriazol-2-yl)-4-methyl-6-(2-methyl-3-(1,3.3,3-tetramethyl-1-(trimethylsilyl)oxi)-disiloxani)propyl) (INCI name: drometrizole trisiloxane); 2-hidroxy-4-methoxibenzofenona-5-sulfonic acid or its sodium salt (INCI name: benzophenone-4 or sulisobenzone); 2,2'-methylen-bis-6-(2H-benzotriazol-2-yl)-4-(tetramethyl-butyl)-1,1,3,3-phenol (INCI name: methylene bis-benzotriazolyl tetramethylbutylphenol); monosodium salt of 2-2'-bis-(1,4-phenylen)1 H-benzimidazol-4,6-disulfonic acid (INCI name: disodium phenyl dibenzimidazole tetrasulfonate); (1,3,5)-triazin-2,4-bis{[4-(2-ethyl-hexyloxy)-2-hidroxy]-phenyl)-6-(4-methoxyphenyl) (INCI name: bis-ethylhexyloxyphenol methoxyphenyl triazine); or 2-(-4-(diethylamino)-2-hidroxybenzoyl)-benzoic acid, hexylester (INCI name: diethylamino hydroxybenzoyl hexyl benzoate). If present, the UVA screen may range from about 0.01% to 20%, preferably, 1 to 10%, more preferably, 2 to 5%, by weight of the total composition.

Preferably, the cosmetic composition used for the purposes of the invention comprises as UVA filters butyl methoxydibenzoyl methane, methylene bis-benzotriazolyl tetramethylbutylphenol, bis-ethylhexyloxyphenol methoxy phenyl triazine, or combinations thereof. Generally, the amount of the butyl methoxydibenzoyl methane is comprised between 1-5% by weight of the total composition. Also, generally, the amounts of methylene bis-benzotriazolyl tetramethylbutylphenol and of bis-ethylhexyloxyphenol methoxy phenyl triazine are comprised between 1-10% by weight of the total composition.

The composition may also comprise one or more UVB screens. The term "UVB screen" means a chemical compound that blocks UV radiation in the wavelength of about 290 to 320 nm. A variety of UVB chemical sunscreens exists and can be used for the purposes of the present invention. Examples of UVB sunscreen are several alpha-cyano-beta,beta-diphenyl acrylic acid esters such as 2-ethylhexyl 2-cyano-3,3-diphenylacrylate (INCI name: octocrylene). Other suitable UVB sunscreens are benzylidene camphor derivatives such as 3-4'-methylbenciliden)-d-l-camphor (INCI name: 4-methylbenzylidene camphor) or 3-benzylidene camphor; cinnamate derivatives such as 2-ethyhexyl 4-methoxycinnamate (INCI name: ethylhexylmethoxy cinnamate); Isopentyl-4-methoxycinnamate (INCI name: isoamyl p-methoxycinnamate); benzophenone derivatives such as oxybenzone or sulisobenzone, or sulisobenzone sodium; salicylate derivatives such as 2-ethyl salicylate (INCI name: ethylhexyl salicylate), 3,3,5-trimethylciclohexyl salicilate (INCI name; homosalate); various aminobenzoic acid derivatives such as p-aminobenzoic acid (INCI name: PABA), ethyl-2-hexyl 4-dimethyl-amino-benzoate (INCI name: ethylhexyl dimethyl PABA), 4,4-((6-(((1,1-dimethylethyl)amino)carbonyl)phenyl)amino) 1,3,5-triazin-2,4-di)diimino)bis-,bis(2-ethylhexyl) benzoate (INCI name: diethylhexyl butamido triazone); drometrizole trisiloxane; bis-ethylhexyloxyphenol methoxy phenyl triazine; or 2,4,6-trianilino-p-(carbo-2'-ethylhexyl-1'oxy)-1,3,5-triazine (INCI name: ethylhexyl triazone). If present, the UVB screen may range from about 0.01% to 20%, preferably, 1 to 10%, more preferably, 2 to 5%, by weight of the total composition.

Preferably, the cosmetic composition used for the purposes of the invention comprises as UVB filters octocrylene, ethylhexyl methoxycinnamate, diethylhexyl butamido triazone or combinations thereof. Generally, octocrylene content is comprised between 1 to 10% by weight of the total composition, preferably between 6-10% by weight of the total composition. Generally, the amount of ethylhexyl methoxycinnamate is comprised between 5-10% by weight of the total composition. Generally, the amount of diethylhexyl butamido triazone is comprised between 1-10% by weight of the total composition.

Preferred screens according to the invention are diethylhexyl butamido triazone, octocrylene, ethylhexyl methoxycinnamate, butyl methoxydibenzoylmethane, diethylamino hydroxybenzoyl hexyl benzoate, or ethylhexyl salicylate.

The compositions used for the purposes of the invention may be formulated to have a certain sun protection factor (SPF) values ranging from about 1-50, preferably 2-45, more preferably 5-30. The SPF indicates the increase of time the skin may exposed to the sun, without suffering adverse effects: flushing, erythema and burns. Calculation of SPF values is well known in the art.

If present, a preferred physical screen according to the invention is titanium dioxide micronized.

If present, preferred organomineral filters include methylene bis-benzotriazolyl tetramethylbutylphenol (tinosorb® m) or bis-ethylhexyloxyphenol methoxyphenyl triazine (tinosorb® s).

If present preferred biological screens are substances with antioxidant activity such as vitamins (A, C or E), flavonoids (Fe chelants) and other oligoelements (enzymatic activity). The most preferred biological filters are vitamins (A, C, or E).

In a particular embodiment, the compositions used for the purposes of the invention comprise the lysate of *bifidobacterium longum* in combination with at least a chemical filter and at least an organomineral filter. In another particular embodiment, the composition used for the purposes of the invention further comprises at least a physical filter. Preferably, the chemical and organomineral filters are selected from those mentioned above.

In another particular embodiment, the compositions used for the purposes of the invention comprise the lysate of *bifidobacterium longum* in combination with at least a chemical filter and at least a physical filter. Preferably, the chemical and physical filters are selected from those mentioned above.

In a preferred embodiment, the composition comprises the lysate of *bifidobacterium longum* in combination with ethylhexyl methoxycinnamate, octocrylene, butyl methoxydibenzoylmethane, diethylhexyl butamido triazone, and bis-ethylhexyloxyphenol methoxyphenyl triazine.

The compositions used for the purposes of the invention comprise additional excipients or carriers appropriate for topical application to a person's skin.

Among these excipients or carriers the following are preferred: moistening agents such as litchi extract; emollient agents; antioxidant agents such as argan extract, vitamin E acetate, or green tea oil; revitalizing agents such as rice protein, boosters of SPF such as wheat proteins derivatives or amino acids with cutaneous compatibility; water resistant polymers; preservatives; emulsifiers; volatile silicones; gelling agents such as xantham gum or sclerotium gum; perfumes; or colorants.

The cosmetic compositions used for the purposes of the invention may be in the form of emulsion, cream, milk, lotion, ointment, solid stick, foam, spray, oil, pomade and fluid, among others. They can be in anhydrous form, in an aqueous solution, in suspension form, or in the water-in-oil or oil-in water emulsion form.

In general any composition used for the purposes of the invention may be applied to the skin, in any parts of the skin, in any part of the body.

The compositions used for the purposes of the present invention become preferred before, during or after an irradiation of the skin with IR.

It is disclosed a method, for preventing and/or treating skin cells from damage by exposure to IR radiation, in particular IRA radiation i.e. cellular damages induced or produced by IRA radiation, comprising administering an effective amount of a lysate of at least a microorganism of the genus bifidobacterium species, in particular *bifidobacterium longum,* together with acceptable carriers or excipients appropriate for topical application to a person's skin, to a subject in need of such prevention and/or treatment. It can be used cosmetically or pharmaceutically acceptable carriers or excipients. More particularly, the skin cell damage is photoaging induced or produces by IRA radiation.

It is also disclosed that the method further comprises preventing and/or treating skin cells from damage by exposure to UV radiation, by administering the lysate of at least a microorganism of the genus bifidobacterium species in combination with at least a filter against solar radiation selected from the group consisting of physical, chemical, organomineral, biological filters, and combinations thereof.

The method comprises a single administration or a repeated administration depending on the time of exposure to sunlight.

In a particular embodiment, the prevention and/or treatment comprises protecting the skin from damages induced or produced by exposure to IR radiation, in particular to IRA radiation.

In a particular embodiment, the prevention and/or treatment comprises diminishing ROS generation directly induced by IRA radiation. In another particular embodiment of the method, the prevention and/or treatment comprises inhibiting the MMP-1 production induced by IRA radiation.

This aspect of the invention and the corresponding embodiments can be formulated as a lysate of at least one microorganism of the genus *Bifidobacterium* species, in particular *Bifidobacterium longum*, for use in the prevention and/or treatment of skin cells damage produced by IR radiation, in particular, IRA radiation. The skin cells damage is the damage that is done to the skin from prolonged exposure, to IRA radiation. More particularly, the skin cell damage is photoaging induced or produced by IR radiation, in particular IRA radiation. It can also be formulated as the use of a lysate of at least one microorganism of the genus *Bifidobacterium* species, in particular *Bifidobacterium longum*, for the preparation of a composition for the prevention and/or treatment of skin cells damage produced by IR radiation, in particular, IRA radiation.

It is also part of the invention the provision a lysate of at least one microorganism of the genus *Bifidobacterium* species, in combination with at least a filter against solar radiation selected from the group consisting of physical, chemical, organomineral, biological filters, and combinations thereof, for use in the prevention and/or treatment of skin cells damage produced by IR radiation, in particular IRA radiation and Ultraviolet radiation. More particularly, the skin cell damage is photoaging induced or produced by IR radiation, in particular IRA radiation and UV radiation. In that case the skin cells damage is the damage that is done to the skin from prolonged exposure, to IR radiation and UV radiation. This aspect can also be formulated as the use of a lysate of at least one microorganism of the genus *Bifidobacterium* species, in particular *Bifidobacterium longum*, for the preparation of a composition for the prevention and/or treatment of skin cells damage produced by IR radiation, in particular, IRA radiation and UV radiation.

In a preferred embodiment, the lysate of at least one microorganism is a lysate of *Bifidobacterium longum*.

In another preferred embodiment, the use is for prevention of skin cells damage.

In another preferred embodiment, the effective amount of the lysate is comprised between 0.1-5% in weight based on the total weight of a composition comprising it. In a more preferred embodiment, the effective amount of the lysate is comprised between 0.5-3% in weight based on the total weight of a composition comprising it. In a still more preferred embodiment, the effective amount of the lysate is comprised between 0.5-1% in weight based on the total weight of a composition comprising it.
It is also considered part of the invention a method for diminishing ROS generation directly induced by IRA radiation, comprising administering an effective amount of a lysate of at least a microorganism of the genus bifidobacterium species, in particular *bifidobacterium longum*, together with cosmetically acceptable carriers or excipients, to a subject in need of such prevention and/or treatment.

Finally, it is also disclosed a method for inhibiting the MMP-1 production induced by IRA radiation, comprising administering an effective amount of a lysate of at least a microorganism of the genus bifidobacterium species, in particular *bifidobacterium longum*, together with cosmetically acceptable carriers or excipients, to a subject in need of such prevention and/or treatment.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

Repair Complex PF or RCPF is used indistinctly to denote the compound Repair Complex paraben free.

### Example 1: Facial cream dry skin SPF 20

The following ingredients were used:

| Ingredient code | INCI name | % in weight |
|---|---|---|
| 1 | Ethylhexyl salicylate | 5 |
| 2 | Octocrylene | 10 |
| 3 | Butyl methoxydibenzoylmethane | 4 |
| 4 | Ethylhexyl methoxycinnamate | 8 |
| 5 | Dibutyl adipate | 3 |
| 6 | Hydrogenated polyisobutene | 2 |
| 7 | Diethylhexyl butamido triazone | 3 |
| 8 | Bis-ethylhexyloxyphenol methoxyphenyl triazine | 2,5 |
| 9 | Cetearyl alcohol (and) cocoglucoside | 1 |
| 10 | C₂₀-₂₂ alcohols | 3 |
| | C₂₀-₂₂ alkyl phosphate | |
| 11 | VP/Eicosene copolymer | 2,5 |
| 12 | Aqua (Water) | 47,025 |
| 13 | Sclerotium gum | 4 |
| | Aqua (Water) | |
| | Phenoxyethanol | |
| 14 | Aqua (Water) | 0,5 |
| | Pentasodium ethylene diamine tetramethylene phosphonate | |
| 15 | Tromethamine | 0,375 |
| 16 | Ethylhexylglycerin | 1 |
| | Phenoxyethanol | |
| 17 | Bifida Ferment Lysate | 1 |
| | Phenoxyethanol | |
| | Sodium benzoate | |
| 18 | Perfume | 0,1 |
| 19 | EDTA | 2 |
| | Ethylhexylglycerin | |
| | Potassium sorbate | |
| | Hydrolyzed wheat protein / PVP crosspolymer | |
| | Phenoxyethanol | |
| | Aqua(Water) | |
| | Total: | 100 |

When one ingredient code have more than one component is because of this ingredient is a mixture of several components.

The preparation of the composition was effected by combining ingredients 1-6 at 70 ° C ± 10 °C. Subsequently, ingredient 7 was added under stirring, and then ingredient 8 was added also under stirring. Once a transparent solution was obtained, ingredients 9 and 10 were added. Once a transparent solution was obtained ingredient 11 was added. Ingredient 12 (water) was heated at 70 ° C ± 10 °C and ingredient 13 was added under stirring. To this aqueous phase ingredients 14 and 15 were added. Then both phases were homogenized and cooled to 40 °C. At this temperature ingredients 16, 17, 18, and 19 were added.

### Example 2: Evaluation of the efficacy of the product Repair Complex PF (RCPF) as a protector against IRA radiation

A reproduction of the cellular situation induced by IRA was carried out by inducing ROS linked to the increase of the cellular temperature. It is known that there is a proportional relation between the exposition time to IRA radiation and the increase in ROS generation. The greater the exposure to IRA, the greater the increase in skin temperature and of ROS generation. Thus, it was carried out an *in vitro* simulation of the thermal stress conditions analogous to those induced by IRA radiation in the model human dermal fibroblast (HDF). The assay was carried out with human dermal fibroblast at a constant temperature of 42 °C in the presence and absence of the lysate of *bifidobacterium longum* (the commercial product named Repair Complex PF, RCPF) and with control cells maintained at 37 °C.

The protective effect was determined by measuring the ROS intracellular production as a consequence of an indirect effect of the increase of temperature which would be caused by the IRA radiation, and by determining the levels of synthesis of metalloproteinase 1 as a final effect of thermal stress (signal produced by the increase of ROS due to the increase of temperature).

### Equipment:

Fluorescence Microplate Reader (Fluoroskan Ascent CF Thermofisher Labsystems).
ELISA reader (spectrophotometer) Multiskan RC Thermofisher Labsystems. SAPHIRE-2 reader (fluorescence, luminescence and spectrophotometer) of TECAN.

### Cellular model:

As an *in vitro* cellular model have been used primary cultures of human dermal fibroblasts (HDF), obtained from human skin, from one or more (pool) skin samples from phimosis operation in children between 0 and 3 years. These fibroblasts have been characterized and expanded in medium Dulbecco's, 1 g / I glucose supplemented with 10% fetal calf serum (FCS), 2 mM L-glutamine and antibiotics (100 mg / ml Penicillin and 100 U / ml of streptomycin.

### Evaluation of the cellular cytotoxicity in human dermal fibroblasts

The determination of cytotoxicity was performed by the method WST1, a standard colorimetric assay for the non-radioactive quantification of cell proliferation, cell viability, and cytotoxicity. This assay is based on the reduction of a tetrazolium salt by mitochondrial reductases action, forming a soluble colored product whose concentration is determined by measuring the optical density at 450 nm. High values are indicative of healthy cells, while low values are indicative of cytotoxic damage.

Briefly, at 24 hours after seeding, the cells were exposed to 6 concentrations (20, 10, 5, 1, 0.5 and 0.25% in test medium) of the suspension RCPF for 24 h and were maintained at 37 °C, and at 42 °C on a 5% CO₂ incubator. After 24 hours of exposure to the product, cell culture medium was decanted, and WST1 reagent was added directly into the culture medium in the absence of product. After 1 and 2 hours of development, the optical density or absorbance was read with an ELISA reader 450 nm wavelength.

The product assayed Repair complex does not show any toxic effect within the range 20, 10, 5, 1, 0,5 and 0,25 % in fibroblast.

### Determination of the effect of RCPF over intracellular anti-oxidant capacity in HDF cells

The rate of formation of ROS was assessed in cells treated and cells not treated, and kept at 37 °C (control) and 42 °C (IRA effect) for different times (60, 120, 180, and 240 minutes), using the probe 2,7-di-chloro-di-hydro-fluorescein di-acetate (Carboxy-H₂DCFDA). C-400 from Molecular Probes. This is a reliable fluorogenic marker for ROS in live cells, sensitive to reactive oxygen species (ROS), which allows to obtain a quantifiable measure of intracellular ROS production and thus to study the efficacy of compounds with supposed anti-radical and antioxidant activity. This probe is an indicator of intracellular ROS production, which remains in state nonfluorescent until oxidation occurs within the cell, when its acetate groups are removed by intracellular esterases.

The rate of formation of ROS at different times were measured in a plate fluorimeter after 40 minutes of incubation of cells with carboxy-H₂DCFDA probe in the presence of product (RCPF), and subsequent activation of the cells, usually with 250 µM tert-butylhydroperoxide (TBHP), also in presence of the product. In the present study the rate of ROS formation was also measured using as stimuli the exposure of the cells at a temperature of 42 °C (thermal stress) for different times (0, 60 min, 120 min, 180 min, and 240 min). This had been compared with the formation of ROS produced in a control plate held in identical control conditions but at 37 °C. In both cases TBHP was used as a positive assay control.

Day 0 (seeding): fibroblasts were seeded in the central part of 96-well plates at a density of 5,000 cells / well (48 wells / plate). A different cell plate was prepared for each study incubation time at 42 °C (60 min, 120 min, 180 min, and 240 min) and a fifth cell plate was prepared to perform the experiment in parallel at 37 °C. These 5 plates with cells were left during 2-3 days in a 5% CO₂ incubator at 37 °C, , until they formed a cell monolayer (80-90% confluence) and before starting the treatment.

Day 3 (treatment): There were prepared 5 plates without cells but identically configured as the previous one, which were named as transition plates: P-1 (1 h, 42 °C), P-2 (2 h, 42 °C), P3 (3 h, 42 ° C), P-4 (4 h, 42 ° C) and P-5 (37 ° C). In these plates, the products of the study were added at a concentration 2X (2 times concentrated) of the desired final concentrations (0.1 %, 1%, and 5% for RCPF and 100 µM for α-tocopherol analogue and reference antioxidant TroloxTM) in Hank's buffered salt solution (HBSS buffer) or the buffer to the wells not treated with compound.

The transition plates allow a more easily transfer of 50 µl of the compounds at 2X or the buffer (control) to the corresponding cell plate, where the probe previously prepared at 2X (40 µM in HBSS buffer) has been previously added to reach then a final concentration of 20µM as follows

The medium of one of the plates with cells was decanted and 50 µl of the probe 2X in buffer were added to all wells, with the exception of the negative control (ctr -). Then, 50 µl of the corresponding transition plate containing the RCPF , Trolox and the controls at 2X were added and incubated 40 minutes at 37 °C to incorporate the probe into the cells. After this labeling time, the plate of the labeled cells was decanted on absorbent paper, washed and added back the compound RCPF and the controls (50µl of the same transition plate). Immediately, TBHP was added only to the wells of the positive control. It was made a reading of the fluorescence at T = 0 and immediately it was started the stimulation moving the plate to the temperature that corresponds (42 ° C for the plates P-1 to P-4) or 37 °C (for P-5).

Quantification of ROS: From each plate ROS production was quantified by fluorometric analysis by measuring the mean fluorescence intensity (MFI), in a Fluorescence Microplate Reader (Fluoroskan Ascent CF; Labsystem) at time 0, and at time corresponding to 60, 120, 180, and 240 minutes at wavelengths of 485 and 527 nm of excitation and emission, respectively. It was read the fluorescence of the transition plate which was then subtracted from all readings as a target to obtain the specific fluorescence (MFI-control).

The results in this case of thermal stress were expressed as increase over the probe and positive control at 37 °C and as % of control medium with the probe and without product of each time and plaque.

The result of MFI was compared to the pro-oxidant compound tert-butyl hydroperoxide (TBHP), used as positive control, and with the probe control without treatment of the same plate and time.

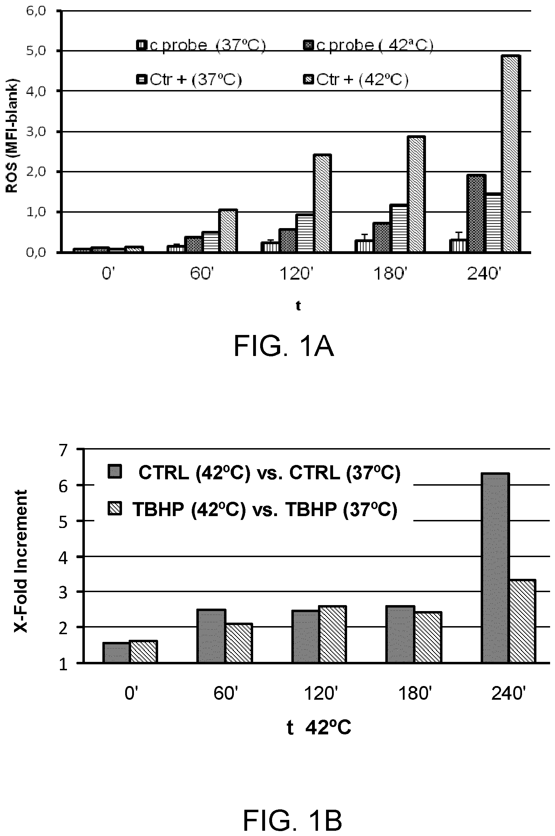

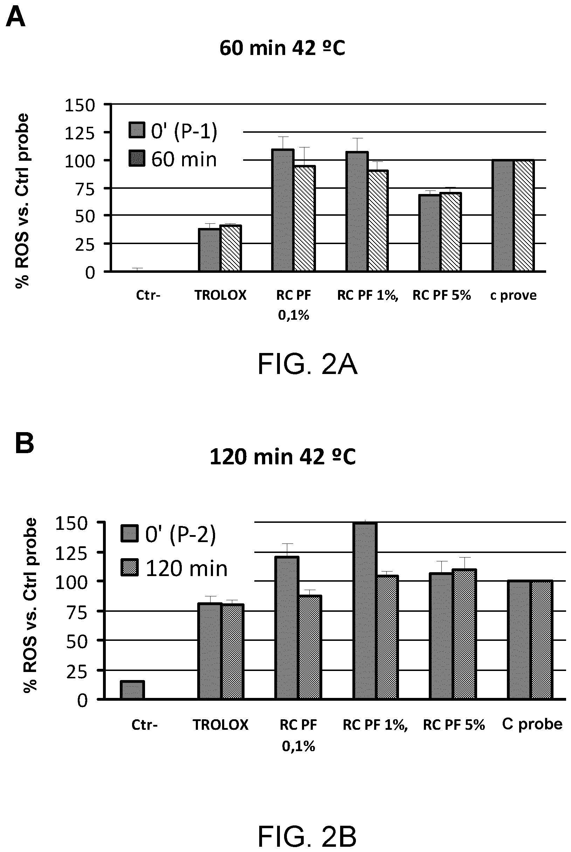

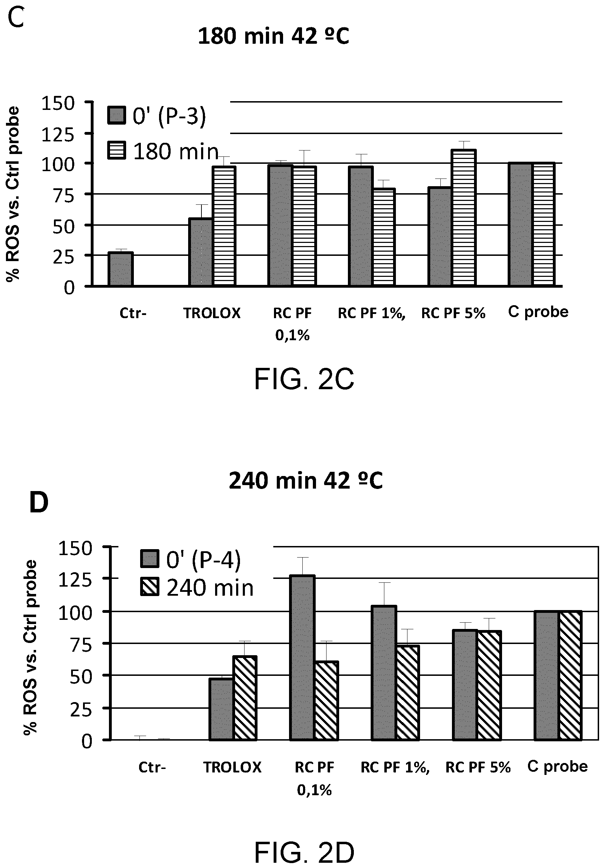

The results are summarized in Table 1 and illustrated graphically in FIG. 1-FIG. 3. Table 1 shows the relative increase of ROS at 42 °C (IRA effect) and the % of inhibition or of increase with respect to the prove control.

**Table 1:**

| | Probe control | | RCPF 0.1% | | | RCPF 1 % | | | |
|---|---|---|---|---|---|---|---|---|---|
| IRA time exposure | 42 °C | | 42 °C | % Ctrl | % inhib. | 42 °C | % Ctrl | % inhib. | |
| 60 min | 3.08 | 100% | 2.44 | 79.3 | 20.7 | 2.57 | 83.6 | 16.4 | |
| 120 min | 4.75 | 100% | 3.59 | 75.7 | 24.3 | 4.63 | 97.6 | 2.4 | |
| 180 min | 6.02 | 100% | 4.42 | 73.3 | 26.7 | 4.03 | 66.9 | 33.1 | |
| 240 min | 15.63 | 100% | 9.66 | 61.8 | 38.2 | 10.84 | 69.4 | 30.6 | |
| | | | | | | | | | |

| | RPCF 5% | | | TROLOX | | | TBHP Ctrl + | | |
|---|---|---|---|---|---|---|---|---|---|
| IRA time exposure | 42 °C | % Ctrl | % inhib | 42 °C | % Ctrl | % inhib | 42 °C | % Ctrl | % inhib |
| 60 min | 3.16 | 102.7 | -2.7 | 3.3 | 107.9 | -7.9 | 8.33 | 271 | -170.8 |
| 120 min | 7.60 | 160.1 | -60.1 | 10.1 | 212.1 | -112.1 | 18.87 | 398 | -297.8 |
| 180 min | 8.77 | 145.5 | -45.5 | 14.0 | 232.8 | -132.8 | 22.40 | 372 | -271.8 |
| 240 min | 19.46 | 124.6 | -24.6 | 26.8 | 171.3 | -71.3 | 38.09 | 244 | -143.8 |

These results show that the thermal stress (fixed temperature at 42°C) produced an increase in intracellular ROS production over time compared with the control at 37 °C and with the values of TBHP (positive control of ROS assay). Under these conditions, the product RCPF protects of the direct ROS increase induced by temperature (IRA effect) over time.

At 0.1% RCPF the inhibitory effect is time dependent and maximum at 240 minutes (38% inhibition). An inhibitory effect of the same order (30-33%) was also observed at 1% but only after 180 and 240 minutes of thermal stress. The classic antioxidant Trolox does not have any protective effect at long times, and the positive control (TBHP) increases with time the control response by 171%, 298%, 272% and 144% at different times post stress.

To sum up, the results show that the RCPF before a temperature increase induced by IRA, protects of the direct ROS increase over time.

### Determination of the effect of RCPF over of the synthesis of metalloproteinase-1 (MMP-1) in HDF cells

The synthesis of MMP-I (ng / mg protein) was measured by ELISA in the culture medium after maintaining the cells at 42 °C (IRA effect) for different times (60, 120, 180 and 240 minutes) and after incubating them for 24 h, with or without the treatments.

Determination of the product (RCPF) effect on the regulation of MMP-1 was performed using a commercial kit from Biotrack for measuring the total synthesis and activity of metalloproteinase-I (MMP-1), one of the main skin metalloproteinases, responsible for the degradation of collagen type I and type III, among others.

It was assessed the protective effect of the product in cells maintained at 42 °C at different incubation times before performing the ELISA and the quantification according to the KIT indications. (See below)

For these assays cells were seeded in 24 wells culture plates at 30,000 cells / well and after 48 hours of growth, one plate for time (1 h, 2 h, 3 h, and 4 h) was incubated at 42 °C with growth complete medium and one additional plate was remained at 37 °C. The compounds were applied (0.5 ml well) prepared at the indicated concentrations in assay medium Dulbeco's modified Eagles medium (DMEM) 1 g / I with 1% fetal calf serum (FCS) in triplicate as indicated in the plate scheme below and kept for 24 hours at 37 °C (Table 2), before MMP-1 quantification

It was used the IL-1β at 5ng/ml as a known activator of the synthesis of MMP-1. It was determined if the product RCPF, at a concentration (1 %) was able to inhibit the synthesis of MMP -1 induced by an inflammatory stimulus to the skin such as IL-1β. As a positive control was used dexamethasone (DEXA) 1 µM to validate the assay.

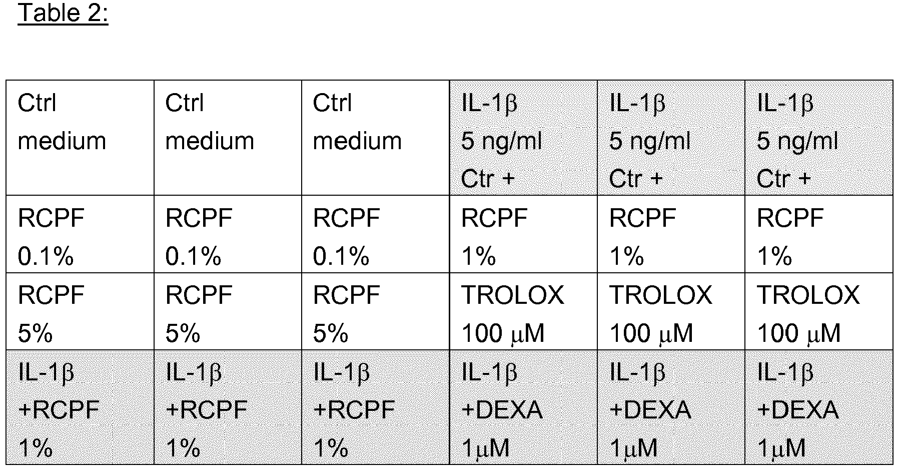

Grey cells means wells inflamed with 1L-1β (5ng/ml) alone and combined with the mentioned compounds. CTRL in the previous table means control medium.

After the incubation time, 24 hours at 37 °C, the culture medium was collected and several aliquots were made and frozen at -80 °C until the amount of MMP-1 synthesized per well was measured. The measurement was made in duplicate using a commercial kit BIOTRACK for the measurement of MMP-1, according to the manufacturer's instructions. In parallel, plates were frozen at - 80 °C and then cell lysates were made from each well. The amount of total protein in the wells of the assay was determined by the BCA Protein Assay Reagent Kit from Pierce. The value of the quantification of MMP-1 in ng / ml was corrected for the total protein in each experimental condition, in this way the value of MMP-1 ng / total protein was obtained.

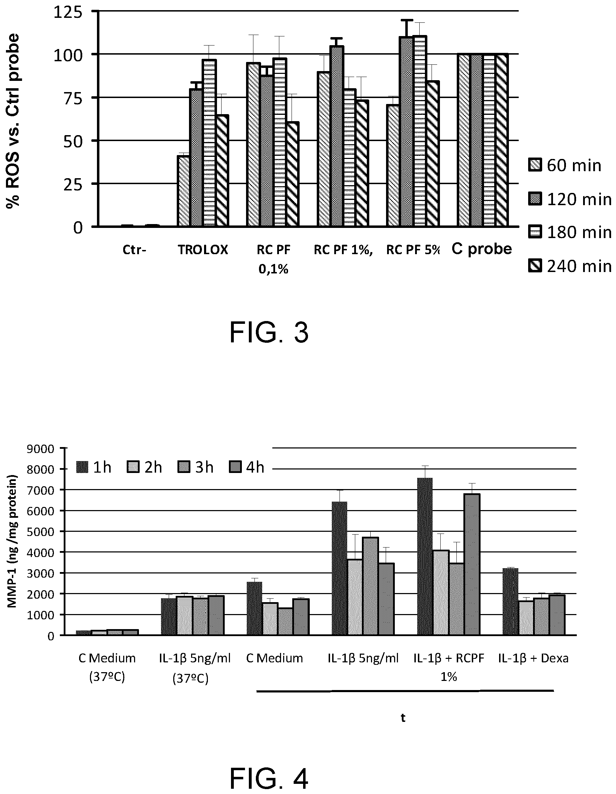

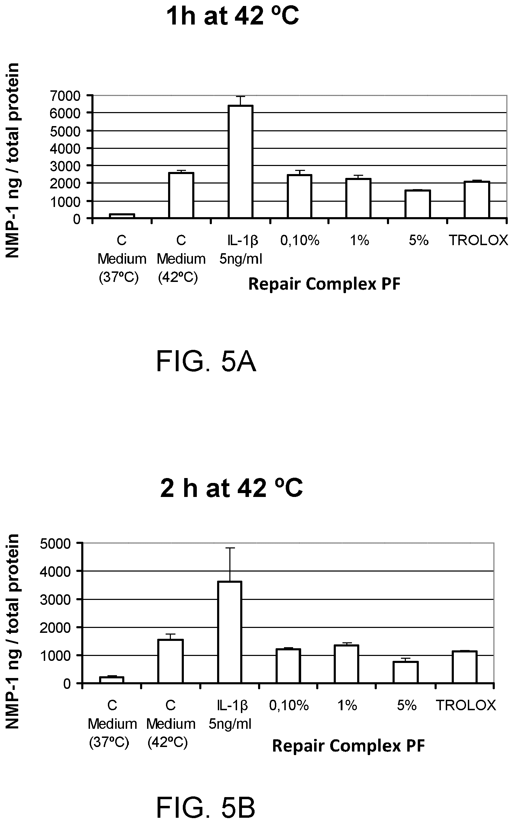

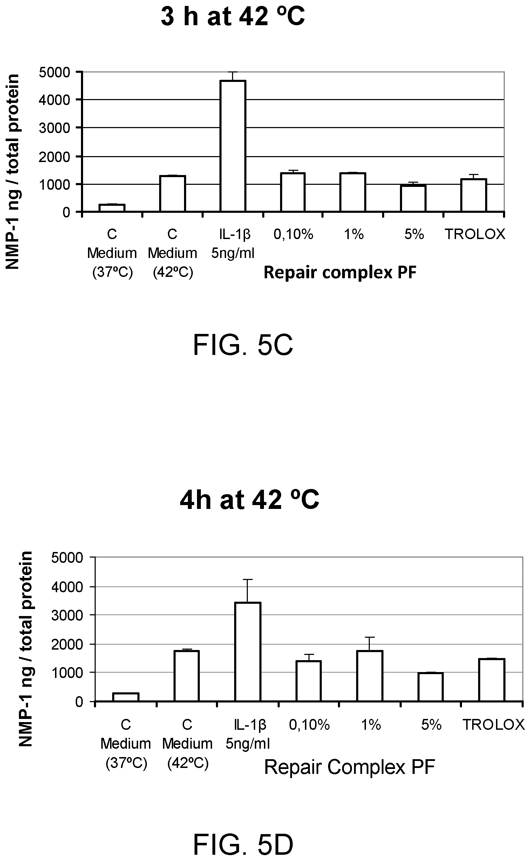

The results are summarized in FIG. 4 and FIG. 5A-D. The results show that the synthesis of MMP-1 was increased at 24 h after incubation at 42 °C for 60 min at a maximum of 12 times (2571.03 ± 165.4) when compared to basal values of MMP-1 at 37 °C (≈ 216 ± 18 , 6 ng / ml) and about 3.5 times when compared with the effect of 5 ng / ml of IL-1β (6422.8 ± 537.6). For the remaining times of incubation, the increase was lower and similar at 120 min, 180 min, and 240 min (≈ 8 - vs. ≈ 6.5 times, control at 37 °C). Under these conditions, the product RCPF inhibited by 10% and 50% the synthesis of MMP-1 induced during 24 h in cells previously incubated at 60,120,180, and 240 minutes at a temperature of 42°C. The effect was dose dependent only for short periods (60 and 120 minutes at 42 ° C) and when the synthesis of MMP-1 of the control has been greater. At 5% concentration, the inhibition founded varied between 30-50% at 60 and 240 minutes. The inhibition with an antioxidant as Trolox (α-tocopherol) was 20-25% at 60 and 120 minutes. At an intermediate concentration of 1% of the product RCPF does not inhibit the synthesis of MMP-1 induced by IL-1β (5 ng / ml) and even enhances the effect this cytokine, both short times of temperature (h at 42 °C + 24 h at 37 °C in the presence of product(RCPF)) and a longer times 4 h.

To sum up, the results show that the RCPF before a temperature increase induced by IRA protects of the increase of the MMP-1.

### REFERENCES CITED IN THE APPLICATION

- EP43128
- US4464362
- EP2233127
- WO2009/0535642
- US20090060962
- US20090068610
- Zastrow L. et al. "The Missing Link - Light-Induced (280-1,600nm) Free Radical Formation in Human Skin ", Skin Pharmacol and Physiol 2009, vol. 22, pp. 31-44
- Darvin M.E. et al. "Radical Production by Infrared A Irradiation in Human Tissue", Skin Pharmacol and Physiol 2010, vol. 23, pp. 40-46).
- P. Schroeder et al., "Cellular response to infrared radiation involves retrograde mitochondrial signaling", Free Radic. Biol. Med. 2007, vol. 43, pp. 128-135
- Council of the European Communities (revised text of European Directive 76/768/EEC consolidated version Annex VII pages 121-126 , published on 24.04.2008

## Claims

1. An effective amount of a lysate of at least one microorganism of the genus *Bifidobacterium* species, for use in the prevention and/or treatment of the cutaneous signs of photoaging induced or produced by Infrared A radiation.

2. The lysate as defined claim 1, in combination with at least a filter against solar radiation selected from the group consisting of physical, chemical, organomineral, biological filters, and combinations thereof, for use in the prevention and/or treatment of the cutaneous signs of photoaging induced or produced by Infrared A radiation and Ultraviolet radiation.

3. The lysate for use according to any of the claims 1-2, wherein the lysate of at least one microorganism is a lysate of *Bifidobacterium longum*.

4. The lysate for use according to any of the claims 1-3, wherein the use is for prevention of the cutaneous signs of photoaging.

5. The lysate for use according to any of the claims 1-4, wherein an effective amount of the lysate is used, being comprised between 0.1-5% in weight based on the total weight of a cosmetic composition comprising it.

6. The lysate for use according to claim 5, wherein the effective amount of the lysate is comprised between 0.5-3% in weight based on the total weight of a cosmetic composition comprising it.

7. The lysate for use according to claim 6, wherein the effective amount of the lysate is comprised between 0.5-1 % in weight based on the total weight of a cosmetic composition comprising it.

8. A topical cosmetic composition comprising a lysate of at least one microorganism of the genus *bifidobacterium* species, and at least a filter against solar radiation selected from the group consisting of physical, chemical, organomineral, biological filters, and combinations thereof in an amount of between 29-50% by weight of the total composition, together with cosmetically acceptable excipients or carriers appropriate for topical application to a person's skin, for use in the prevention and/or treatment of the cutaneous signs of photoaging induced or produced by Infrared A radiation and Ultraviolet radiation.

9. The composition for use according to claim 8, consisting of a lysate of at least one microorganism of the genus *bifidobacterium* species, and at least a filter against solar radiation selected from the group consisting of physical, chemical, organomineral, biological filters, and combinations thereof in an amount of between 29-50% by weight of the total composition, together with cosmetically acceptable excipients or carriers appropriate for topical application to a person's skin.

10. A lysate of at least one microorganism of the genus *Bifidobacterium* species, for use in the prevention and/or treatment of skin cells damage produced by Infrarred A radiation.

11. The lysate for use according to claim 10, which is a lysate of *Bifidobacterium longum*.

12. The lysate for use according to any of the claims 10-11, wherein the prevention and/or treatment comprise diminishing reactive oxygen species generation directly induced by Infrared A radiation.

13. The lysate for use according to any of the claims 10-12, wherein the prevention and/or treatment comprises inhibiting the matrix metalloproteinase-1 production induced by Infrarred A radiation.

14. The lysate for use according to any of the claims 10-13, wherein the lysate is in combination with at least a filter against solar radiation selected from the group consisting of physical, chemical, organomineral, biological filters, and combinations thereof, and the combination is further for use in the prevention and/or treatment of skin cells damage produced by Ultraviolet radiation.

15. The lysate for use according to any of the claims 10-14, wherein the skin cells damage is photoaging.

## Patentansprüche

1. Eine wirksame Menge eines Lysats von mindestens einem Mikroorganismus der Gattung *Bifidobacterium* zur Verwendung in der Prävention und/oder Behandlung der durch Infrarotstrahlung A induzierten oder verursachten Hautveränderungen, die auf Photoaging hinweisen.

2. Das Lysat wie in Anspruch 1 definiert, in Kombination mit mindestens einem Filter gegen Sonnenstrahlung, der aus der Gruppe bestehend aus physikalischen, chemischen, organomineralen, biologischen Filtern, und Kombinationen davon, ausgewählt ist, zur Verwendung in der Prävention und/oder Behandlung der durch Infrarotstrahlung A und Ultraviolettstrahlung induzierten oder verursachten Hautveränderungen, die auf Photoaging hinweisen.

3. Das Lysat zur Verwendung nach einem der Ansprüche 1-2, wobei das Lysat von mindestens einem Mikroorganismus ein Lysat von *Bifidobacterium longum* ist.

4. Das Lysat zur Verwendung nach einem der Ansprüche 1-3, wobei die Verwendung zur Prävention der auf Photoaging hinweisenden Hautveränderungen ist.

5. Das Lysat zur Verwendung nach einem der Ansprüche 1-4, wobei eine wirksame Menge des Lysats verwendet wird, die bei zwischen 0,1-5 Gew.-% bezogen auf das gesamte Gewicht einer kosmetischen Zusammensetzung liegt, die es beinhaltet.

6. Das Lysat zur Verwendung nach Anspruch 5, wobei eine wirksame Menge des Lysats bei zwischen 0,5-3 Gew.-% bezogen auf das gesamte Gewicht einer kosmetischen Zusammensetzung liegt, die es beinhaltet.

7. Das Lysat zur Verwendung nach Anspruch 6, wobei eine wirksame Menge des Lysats bei zwischen 0,5-1 Gew.-% bezogen auf das gesamte Gewicht einer kosmetischen Zusammensetzung liegt, die es beinhaltet.

8. Eine topische kosmetische Zusammensetzung umfassend ein Lysat von mindestens einem Mikroorganismus der Gattung *bifidobacterium*, und mindestens einen Filter gegen Sonnenstrahlung, der aus der Gruppe bestehend aus physikalischen, chemischen, organomineralen, biologischen Filtern, und Kombinationen davon ausgewählt ist, in einer Menge von zwischen 29-50 Gew.-% der gesamten Zusammensetzung, zusammen mit kosmetisch akzeptablen Hilfsstoffen oder Trägerstoffen, die geeignet zur topischen Anwendung auf der menschlichen Haut sind, zur Verwendung in der Prävention und/oder Behandlung von durch Infrarotstrahlung A und Ultraviolettstrahlung induzierten oder verursachten Hautveränderungen, die auf Photoaging hinweisen.

9. Die Zusammensetzung zur Verwendung nach Anspruch 8, bestehend aus einem Lysat von mindestens einem Mikroorganismus der Gattung *bifidobacterium*, und mindestens einen Filter gegen Sonnenstrahlung, der aus der Gruppe bestehend aus physikalischen, chemischen, organomineralen, biologischen Filtern, und Kombinationen davon ausgewählt ist, in einer Menge von zwischen 29-50 Gew.-% der gesamten Zusammensetzung, zusammen mit kosmetisch akzeptablen Hilfsstoffen oder Trägerstoffen, die geeignet zur topischen Anwendung auf der menschlichen Haut sind.

10. Ein Lysat von mindestens einem Mikroorganismus der Gattung *Bifidobacterium* zur Verwendung in der Prävention und/oder Behandlung von durch Infrarotstrahlung A verursachter Hautzellschädigung.

11. Das Lysat zur Verwendung nach Anspruch 10, welches ein Lysat von *Bifidobacterium longum* ist.

12. Das Lysat zur Verwendung nach einem der Ansprüche 10-11, wobei die Prävention und/oder Behandlung die Reduktion der Entstehung von reaktiven Sauerstoffspezies umfasst, die unmittelbar durch Infrarotstrahlung A induziert ist.

13. Das Lysat zur Verwendung nach einem der Ansprüche 10-12, wobei die Prävention und/oder Behandlung die Hemmung der durch Infrarotstrahlung A induzierten Produktion von Matrix-Metalloprotease-1 umfasst.

14. Das Lysat zur Verwendung nach einem der Ansprüche 10-13, wobei das Lysat in Kombination mit mindestens einem Filter gegen Sonnenstrahlung steht, der aus der Gruppe bestehend aus physikalischen, chemischen, organomineralen, biologischen Filtern, und Kombinationen davon, ausgewählt ist, und die Kombination ferner zur Verwendung in der Prävention und/oder Behandlung von durch Ultraviolettstrahlung verursachter Hautzellschädigung ist.

15. Das Lysat zur Verwendung nach einem der Ansprüche 10-14, wobei die Hautzellschädigung Photoaging ist.

## Revendications

1. Une quantité efficace d'un lysat d'au moins un microorganisme du genre *Bifidobacterium*, pour l'utilisation dans la prévention et/ou le traitement des signes cutanés du photovieillissement induits ou produits par le rayonnement infrarouge A.

2. Le lysat tel que défini dans la revendication 1, en combinaison avec au moins un filtre contre le rayonnement solaire choisi dans le groupe constitué des filtres physiques, chimiques, organomineraux, biologiques, et des combinaisons de ceux-ci, pour l'utilisation dans la prévention et/ou le traitement des signes cutanés du photovieillissement induits ou produits par le rayonnement infrarouge A et le rayonnement ultraviolet.

3. Le lysat pour l'utilisation selon l'une quelconque des revendications 1-2, dans lequel le lysat d'au moins un microorganisme est un lysat de *Bifidobacterium longum*.

4. Le lysat pour l'utilisation selon l'une quelconque des revendications 1-3, dans lequel l'utilisation est pour la prévention des signes cutanés du photovieillissement.

5. Le lysat pour l'utilisation selon l'une quelconque des revendications 1-4, dans lequel on utilise une quantité efficace du lysat comprise entre 0,1-5% en poids par rapport au poids total d'une composition cosmétique comprenant celui-ci.

6. Le lysat pour l'utilisation selon la revendication 5, dans lequel la quantité efficace du lysat est comprise entre 0,5-3% en poids par rapport au poids total d'une composition cosmétique comprenant celui-ci.

7. Le lysat pour l'utilisation selon la revendication 6, dans lequel la quantité efficace du lysat est comprise entre 0,5-1% en poids par rapport au poids total d'une composition cosmétique comprenant celui-ci.

8. Une composition cosmétique topique comprenant un lysat d'au moins un microorganisme du genre *bifidobacterium*, et au moins un filtre contre le rayonnement solaire choisi dans le groupe constitué des filtres physiques, chimiques, organomineraux, biologiques, et des combinaisons de ceux-ci, dans une quantité d'entre 29-50% en poids par rapport à la composition totale, conjointement avec des excipients ou des véhicules cosmétiquement acceptables appropriés pour l'application topique à la peau d'une personne, pour l'utilisation dans la prévention et/ou le traitement des signes cutanés de photovieillissement induits ou produits par le rayonnement infrarouge A et le rayonnement ultraviolet.

9. La composition pour l'utilisation selon la revendication 8, constituée d'un lysat d'au moins un microorganisme du genre *bifidobacterium*, et au moins un filtre contre le rayonnement solaire choisi dans le groupe constitué des filtres physiques, chimiques, organomineraux, biologiques, et des combinaisons de ceux-ci, dans une quantité d'entre 29-50% en poids par rapport à la composition totale, conjointement avec des excipients ou des véhicules cosmétiquement acceptables appropriés pour l'application topique à la peau d'une personne.

10. Un lysat d'au moins un microorganisme du genre *Bifidobacterium*, pour l'utilisation dans la prévention et/ou le traitement du dommage cellulaire produit par le rayonnement infrarouge A.

11. Le lysat pour l'utilisation selon la revendication 10, qui es un lysat de *Bifidobacterium longum*.

12. Le lysat pour l'utilisation selon l'une quelconque des revendications 10-11, dans lequel la prévention et/ou le traitement comprend diminuer la génération de dérivés réactifs de l'oxygène induites directement par le rayonnement infrarouge A.

13. Le lysat pour l'utilisation selon l'une quelconque des revendications 10-12, dans lequel la prévention et/ou le traitement comprend inhiber la production de métalloprotéinase matricielle-1 induite par le rayonnement infrarouge A.

14. Le lysat pour l'utilisation selon l'une quelconque des revendications 10-13, dans lequel le lysat est combiné avec au moins un filtre contre le rayonnement solaire choisi dans le groupe constitué des filtres physiques, chimiques, organomineraux, biologiques, et des combinaisons de ceux-ci, et la combinaison est en outre pour l'utilisation dans la prévention et/ou le traitement du dommage des cellules de la peau produit par le rayonnement ultraviolet.

15. Le lysat pour l'utilisation selon l'une quelconque des revendications 10-14, dans lequel le dommage des cellules de la peau est le photovieillissement.
